# EUROPEAN PATENT APPLICATION

(11) **EP 2 650 371 A2**
(43) Date of publication of application: **16.10.2013**
(21) Application number: 13159824.5
(22) Date of filing: 18.03.2013
(51) Int. Cl.: C12P 7/56, C12P 7/48, C12P 17/12, C12P 17/18, C12P 21/00, C12P 1/02, C12P 39/00, C07K 14/325, C12N 9/26, A01N 63/04, C12R 1/645, C12R 1/67, C12R 1/685, C12R 1/69, C12R 1/845

(54) **Method for the microbial production of organic acids, other molecules and bioactive substances from bioplastic materials**

(30) Priority: 16.03.2012 IT BO20120137
(71) Applicant: Riff 98 Societa' Cooperativa, Bologna (IT)
(72) Inventor: Accinelli, Cesare, 40100 BOLOGNA (IT)
(74) Representative: Boggio, Luigi

(57) **Abstract**

Method for the production of substances from bioplastic; according to the method, the material made of bioplastic is used by a fungus Rhizopus oryzae and subsequently by an entomopathogenic bacterium Bacillus thuringiensis subsq. Kurstaki so as to obtain CrylAb/CrylAc toxins.

## Description

### TECHNICAL FIELD

The present invention relates to a method for microbial production from bioplastic materials.

In particular, the present invention relates to a method for the use of bioplastic materials, such as bags, bottles and other articles (toys), to obtain, via microbiological processes, molecules and compounds of interest for the industrial, food and biomedical sectors and microbiological preparations for industry and agriculture.

### CONTEXT OF THE INVENTION

Currently the need for environmental sustainability has stimulated and promoted the development of new technologies for the production of consumer goods made from renewable sources. It has been ascertained, in fact, that due to their high environmental persistence and dependence on non-renewable sources, plastic materials and the products obtained from them are increasingly frequently replaced by bioplastic materials. Although an exact definition of the term bioplastic does not exist, the term refers to materials with thermoplastic characteristics, produced from renewable sources and also having, in the majority of cases, characteristics of rapid biodegradability in the environment. There are numerous bioplastic-based products currently on sale, including both products for domestic use, such as bags, plates, cups, bottles, toys, etc., and products for industrial and agricultural use, such as interior panelling for the automotive industry, electronics, mulch films, etc. In some cases the process of replacement of the various plastic products with bioplastic products is taking place rapidly and intensely. In some countries, and specifically in the case of plastic bags, the process has been made obligatory by legislative measures. Although in the majority of cases bioplastic-based products are in environmental terms more compatible with the environment than those made of plastic, they also have drawbacks connected with their production and disposal. In the production phase the bioplastics made from agricultural products can take land away from the cultivation of food crops. Another drawback, common to plastic bags, or bags made of polyethylene and analogous polymers, is the dispersion of said products in the environment and above all in the sea. Although some studies have highlighted their biodegradability also in seawater [see publications by Iman, Gordon, Shogren, Tosteson, Govind, Green: Degradation of starch-poly(beta-hydroxibutyrate-co-beta-hydroxy valerate) bioplastic in tropical coastal waters. Applied and Environmental Microbiology 1999, 65,431-437 and O'Brine, Thompson: Degradation of plastic carrier bags in the marine environment. Marine Pollution Bullettin 2010, 2279-2283] this degradation takes a long time and does not exclude negative repercussions on the environment. Dispersion on land and in the sea of bioplastic bags or other products also has harmful effects on wildlife, since accidental swallowing of or suffocation by said bags can cause serious injury or death of the animals.

The Italian document BO2010A00007 and other publications [Accinelli, Saccà, Abbas, Zablotowicz, Wilkinson: Use of a granular bioplastic formulation for carrying conidia of a non-aflatoxigenic strain of Aspergillus flavus. Bioresource Technology 2009, 100, 3997-4004. Accinelli, Mencarelli, Saccà, Vicari, Abbas: Managing and monitoring of Aspergillus flavus in corn using bioplastic-based formulations. Crop Protection 2012, 32, 30-35] are known, in which bioplastic is used as a carrier for a fungal strain and to selectively isolate the same fungal strain and other fungi from various types of matrix such as the soil and foodstuffs, using bioplastic as a matrix for the production of baits for these fungi. Furthermore, the replacement of a fungal strain inoculated with another fungus is known from publications [Accinelli, Saccà, Battisson, Mencarelli, Grabic: Removal of oseltamivir (tamiflu) and other selected pharmaceuticals from waste water using a granular bioplastic formulation entrapping propagules of Phanerochaete chrysosporium. Chemosphere 2009, 81, 436-443]. Methods for the use of industrial or agricultural production waste for the microbiological production of substances for industrial and food use, such as lactic acid, are also known. In these cases lactic bacteria and fungi are used, for example Rhizopus oryzae. This fungus is able to grow on waste agricultural products rich in starchy substances.

The object of the present invention is to provide a method for microbial production from bioplastic materials which overcomes, at least partially, the drawbacks of the known art and, at the same time, is easy and inexpensive to implement.

The method subject of the invention helps to solve these and other drawbacks, with the transformation of bioplastic material from refuse or waste into other substances which can be readily used in industry and in agriculture. In recycling, products that have come to the end of their functional life undergo processes to reproduce the same again, whereas in the current invention the bioplastic-based products are used to produce substances or products of another type, therefore giving the process characteristics of environmental sustainability and recovery thereof.

### SUMMARY

According to the present invention a method is provided for microbial production from bioplastic material as claimed in the following independent claims and, preferably, in any one of the claims depending directly or indirectly on the independent claims.

### EMBODIMENTS OF THE INVENTION

The subject of the described result is based on the use of micro organisms for the specific production of molecules, bioactive substances or preparations starting from waste products made from bioplastic materials. The method according to the result therefore uses bioplastic and micro organisms in order to obtain substances having an economic value. The method for the microbial production of organic acids and bioactive substances from bioplastic materials entails essentially the use of micro organisms and material made of bioplastic. Unlike the products made of plastic materials, those made of bioplastic can be used by some micro organisms, for example some fungi, to obtain, by means of the method illustrated below in detail, other substances, such as organic acids and bioactive substances, for use in industry and in agriculture with obvious economic advantages arising not only from elimination of the risk of dispersion in the environment of the bioplastic products, but also from obtaining, from the bioplastics to be disposed of, other materials that can be used in industry and in agriculture.

A method is therefore provided for microbial production. In particular, a method is provided for the microbial production of other substances (more specifically, organic acids, other molecules and bioactive substances) from bioplastic materials, comprising micro organisms and material made of bioplastic.

A material made of bioplastic is used by the fungus Rhizopus oryzae for the production (i.e. to obtain), in particular via a direct fermentation process, other substances, in particular lactic acid. Advantageously, this takes place at a temperature from approximately 20°C to approximately 35°C.

In particular, the material made of bioplastic is chosen from the group consisting of: pulverised bioplastic bottles, fragmented bioplastic bags, in particular with dimensions of less than 1 mm (and a combination thereof).

According to some embodiments, the material made of bioplastic comprises (more precisely, consists of) pulverised bioplastic bottles.

According to some embodiments, the material made of bioplastic comprises (more precisely, consists of) fragmented bioplastic bags (in particular with dimensions of less than 2 mm; more precisely, less than 1 mm).

In particular, the bioplastic is chosen from the group consisting of: polylactic acid (also known as PLA), bioplastic obtained from starch (in particular, Mater-Bi^{®}) (and a combination thereof).

According to some embodiments, the bioplastic comprises (more precisely, consists of) polylactic acid.

According to some embodiments, the bioplastic comprises (more precisely, consists of) bioplastic obtained from starch (in particular, Mater-Bi^{®} and specifically of types or grades pertaining to the one defined as PE01S).

It should be noted that the bioplastic obtained from starch (in particular, Mater-Bi^{®}) is not composed of crystalline starch as present in nature in cereals, tubers and other vegetables. More precisely, for the production of this type of bioplastic, raw materials containing starch are treated so that the starch contained in them is destructured and complexed with synthetic polymers, as is well known from some publications [Bastioli: Global Status of the Production of Biobased Packaging Materials, Starch/Stärke, 2001, 53, 351-355; Bastioli: Properties and applications of Mater-Bi® starch-based materials, Polymer Degradation and Stability, 1998, 59,263-272].

According to some embodiments, Rhizopus oryzae does not comprise (more precisely, is not) Rhizopus oryzae DSM 905. It has been experimentally observed that this strain allows fumaric acid to be obtained (a product of minor interest).

In particular, the fungus Rhizopus oryzae is chosen from the group consisting of: Rhizopus oryzae CCM 8116, Rhizopus oryzae MB1 (and a combination thereof). According to some embodiments, the fungus Rhizopus oryzae comprises (more precisely, is) Rhizopus oryzae CCM 8116.

In some cases, subsequently (i.e. after the fungus Rhizopus oryzae has initiated its action), treatment with the entomopathogenic bacterium Bacillus thuringiensis subsq. Kurstaki is also carried out. Advantageously, this takes place at a temperature from approximately 20°C to approximately 35°C.

More precisely, the substance obtained from utilisation of the bioplastic material by the Rhizopus oryzae is used by the entomopathogenic Bacillus thuringiensis subsq. Kurstaki. In this way, toxins are obtained, in particular toxins Cry1Ab/Cry1Ac (and parasporal crystals).

In particular, it should be noted that growth of the entomopathogenic Bacillus thuringiensis subsq. Kurstaki in the conditions referred to (and therefore the production of toxins and/or parasporal crystals) was completely unexpected and surprising.

Advantageously, the bioplastic comprises (more precisely, is) polylactic acid.

According to some embodiments, the entomopathogenic bacterium Bacillus thuringiensis subsq. Kurstaki comprises (more precisely, is) the entomopathogenic bacterium Bacillus thuringiensis subsq. Kurstaki HD1.

In particular, the present invention refers to the decomposition of bioplastic-based bags and other products in microbiological processes to obtain substances deriving from the metabolism and biomass thereof. More specifically, the method described is based on the use of micro organisms able to use the bioplastic matrices of the various products made of this material which have come to the end of their functional life in order to obtain substances, molecules, metabolites, antibiotics, biomass and other bioactive substances.

In a more specific form, the method subject of the invention entails the use of bioplastic bags fragmented and used by the fungus Rhizopus oryzae CCM 8116 for the production of lactic acid via a direct fermentation process. In another embodiment, the fungus Rhizopus oryzae CCM 8116 is replaced by the fungus Rhizopus oryzae MB1 to obtain lactic acid via a direct fermentation process. In another form of application, the material consists of bioplastic bags used by the fungus Aspergillus niger CCM 8189 for the production of citric acid via a direct fermentation process. In this case the method for microbial production is essentially the same but with a different liquid culture medium. In a further form of application, the method comprises fragmented bioplastic bags used by the fungus Aspergillus flavus CCM F-449 for the production of aflatoxins B and the antibiotic aspergillic acid. In a further embodiment the material consists of bioplastic bottles pulverised and used first by the fungus Rhizopus oryzae CCM 8116 for the production of lactic acid and then by the entomopathogenic bacterium Bacillus thuringiensis subsp. Kurstaki HD1 for the production of an insecticide prepared from parasporal crystals and Cry1Ab/Cry1Ac toxins. The method subject of the result can also be used for fragmented low density bioplastics, used for example to obtain toys (comprising the above toys made of fragmented bioplastic) and used by the fungus Beauveria bassiana DSM 1344 for the production of an entomopathogenic micoinsecticide preparation consisting of biomass of said fungus. The low density bioplastics (used for toys) can also be used to obtain lactic acid. Lastly, the method according to the result comprises commercial articles made of low density bioplastic, for example toys, used by the fungus Aspergillus oryzae ATCC 12892 for production of the enzyme alpha amylase.

According to the method illustrated in different forms of application, the unit of the invention is provided because of essentially equivalent preparation in all its forms.

According to some aspects, a method is provided for the microbial production of organic acids, other molecules and bioactive substances from bioplastic materials, comprising micro organisms and material made of bioplastic, **characterised in that** the material consists of low density bioplastic toys used by the fungus Beauveria bassiana DSM 1344 for the production of an entomopathogenic micoinsecticide preparation consisting of biomass of said fungus.

According to some aspects, a method is provided for the microbial production of organic acids, other molecules and bioactive substances from bioplastic materials, comprising micro organisms and material made of bioplastic, **characterised in that** the material consists of low density bioplastic toys used by the fungus Aspergillus oryzae ATCC 12892 for production of the enzyme alpha amylase.

Unless explicitly specified otherwise, the content of the references (articles, books, patent applications etc.) cited in this text is referred to here in full. In particular the above-mentioned references are incorporated here for reference.

Further characteristics of the present invention will become clear from the following description of some merely illustrative and non-limiting examples.

### Example 1

Use of bioplastic bags for the microbiological production of lactic acid.

After the fragmentation of bioplastic bags of Mater-Bi^{®} type, defined previously, by means of mechanical shredder (model 7009G; Waring Laboratory, Torrington, USA) to obtain fragments having dimensions of less (<) than 2 mm, 2.5 grams (g) of material were transferred to 250 mL flasks containing 50 mL of a minimal liquid culture medium. The components of the minimal liquid culture medium were the following (per litre): (NH₄)₂SO₄ 1.35 g, MgSO₄ 7H₂O 0.15 g, ZnSO₄ 7H₂O 0.04 g, KH₂PO₄ 0.3 g. The flasks containing the fragments of bioplastic and the liquid culture medium were autoclaved at 120°C and 105 kPa for fifteen minutes. After cooling, the inoculation phase was performed, adding 100 µL of an aqueous suspension of spores (10¹⁰ spores/mL) of the fungus Rhizopus oryzae CCM 8116. The spores were obtained by growing the fungus for five/six days at 28°C in Petri dishes containing the solid medium (2% agar) of dextrose and extracts of potato (potato dextrose agar). After the incubation period, the spores were collected, adding to the Petri dishes 10 mL of sterile water and, with the aid of a sterile loop, the dispersion of spores was transferred to a sterile culture tube. The spores were counted by means of hemocytometer (Burker chamber). Once the inoculum had been added, the flasks were closed with a sterile cotton bung and incubated at 28°C in an orbital incubator at 200 revolutions per minute (rpm). After two days of incubation and therefore with lowering of the pH value to approximately 3.4-4.0, caused by the growth and metabolism of the fungus, the initial pH value was restored (approximately 7.0-7.5) with the addition of CaCO₃ (0.12 g per flask) previously autoclaved. After addition of the carbonate, the flasks were incubated for a further five days, at the end of which the lactic acid concentration was analysed by means of high pressure liquid chromatography (HPLC), according to the method described in the following article: Miura, Tomohiro, Minako, Kojima, Dwiarti, Okabe: Scale-up of L-lactic acid production by mutant strain Rhizopus sp MK-96-1196 from 0.003 m(3) to 5 m(3) in airlift bioreactors. Journal Applied Bioscence Bioengineering 2003, 95, 65-69. The analyses were performed using the HPLC instrument series 200 LC model (Perkin Elmer Co., Norwalk, USA) equipped with CLC-ODS column (Shimadzu Ltd., Kyoto, Japan). The process provided a quantity of laevorotatory lactic acid equal to 120 mg per g of bioplastic added. Repeating the process with material crushed more finely (< 1 mm) and with the strain Rhizopus oryzae MB1 (GenBank Accession Number HQ844675.1), the quantity of laevorotatory lactic acid was higher and equal to 205 mg per g of starting material. With further verifications performed by ELISA method (Laccate Dry-Fast kit, Sentinel Diagnostics, Milan, Italy), laevorotatory lactic acid was confirmed in both cases.

### Example 2

Use of bioplastic bags for the microbiological production of citric acid.

The procedure described in example 1 was repeated using the fungus Aspergillus niger CCM 8189 and replacing the minimal culture medium described above with the following (per litre): K₂HPO₄ 1.0 g, NaNO₃ 3.0 g, MgSO₄ 7H₂O 0.5 g, KCl 0.5 g, FeSO₄ 7H₂O 0.01 g. The bioplastic material added (2.5 g) was crushed to obtain dimensions < 1 mm. After six days of incubation at 28°C in an orbital incubator at 200 rpm, the quantities of citric acid produced were 62 mg per g of bioplastic added. The analyses of the concentration of citric acid in the liquid culture medium were performed by means of HPLC according to the method described in the publication: Hang, Woodams: Enzymatic enhancement of citric acid production by Aspergillus niger from corn cobs, Lebensmittel-Wissenschaft und-Technologie 2001, 34, 484-486. The analyses were performed using the series 200 LC model (Perkin Elmer Co., Norwalk, USA) equipped with Aminex HPX-87H column (Bio-Rad Laboratories Inc., Hercules, USA). The separation was performed maintaining the column at a constant temperature of 40°C, as indicated in the above-mentioned method.

### Example 3

Use of bioplastic bags for the microbiological production of aflatoxins B and the antibiotic aspergillic acid.

The procedure described in example 1 was repeated using the fungus Aspergillus flavus NRRL 30796, the culture medium described in example 2 and fragments with dimensions < 2 mm. After six days of incubation at 38°C in an orbital incubator at 200 rpm, the mycelium produced (0.23 g per g of bioplastic added) was separated from the culture broth, centrifuging at 5,000 rpm for five minutes. Portions of the supernatant (3 mL) were transferred into 10 mL centrifuge tubes and then 7 mL of ethanol were added. The tubes were transferred to a horizontal stirrer and left to stir at 100 rpm for ten minutes. The concentration of total B aflatoxins was determined using the ELISA method by means of the AgraQuant® Total Aflatoxin kit produced by Romer Labs, Tulln, Austria. The total B aflatoxins value obtained was 3.4 µg/kg. Repeating the same procedure, but inoculating the flasks with the strain Aspergillus flavus CCM F-449 it was possible to obtain, as confirmed by LC/MS analysis, aspergillic acid, a molecule having antibiotic property.

### Example 4

Use of bioplastic bottles for the sequential microbiological production of lactic acid and bacterial toxins.

Fragments of approximately 4-5 mm² obtained by fragmenting bioplastic bottles (Sant'Anna® mineral water bio bottle) produced with polylactate bioplastic (ingeo^{™}), also called polylactic acid, were transferred to a beaker with volume of 100 mL, containing 20 mL of acetone (to make the bioplastic friable so as to transform the bioplastic into powder). The beakers were then subjected to a temperature of 100°C in a magnetic stirrer for approximately fifteen/twenty minutes. The dispersion obtained was then subjected to a flow of hot air (55°C, 70 m³/hour) and pulverised by a simple electric homogeniser (crusher) (model SS110, Waring Laboratories, Torrington, USA). A quantity of 2 g of the pulverised material was transferred to a 250 mL flask containing 50 mL of the minimal liquid culture medium described in example 1. The flasks were inoculated with Rhizopus oryzae CCM 8116 (100 µL of an aqueous suspension of spores of 10³ spores per mL) and incubated at 28°C in an orbital stirrer at 200 rpm. After one day of incubation, 0.12 g of CaCO₃ were added to buffer the pH, which tends to drop due to the production of lactic acid, and then five days after addition of the carbonate, the lactic acid concentration was determined by means of HPLC according to the method cited in example 1. Before withdrawing the samples for analysis, the liquid phase was separated from the solid phase by centrifugation at 10,000 rpm. The value of the laevorotatory lactic acid produced was 198 mg per g of material (of bioplastic origin) added. As in example 1, an enzymatic assay was performed for verification. After withdrawing portions (3 mL) for analysis, the total content of two flasks was mixed together and then homogenised, again by means of Ultraturrax type electric laboratory homogeniser and then centrifuged again under the same conditions as above. The supernatant (5 mL) was transferred to 250 mL flasks and then inoculated with spores of the bacterium Bacillus thuringiensis subsp. Kurstaki HDI (100 µL of a suspension having 10⁷ colony forming units/mL). After incubation at 28°C for two days, the presence of parasporal crystals was confirmed by phase contrast microscope observations and the concentration of toxins having insecticidal properties was determined by ELISA Bt-CrylAb/CrylAc kit produced by Abraxis, Warminster, USA. Since the concentration of toxins (usable as insecticide) is higher than the maximum value of the standard contained in the kit, successive dilutions were performed on the sample. The estimated value was 810 µg/mL.

### Example 5

Use of bioplastic toy items (made of Mater-Bi^{®} type bioplastic having porous consistency and low density) for the production of biomass of the micoinsecticide preparation Beauveria bassiana.

After mechanical fragmentation of toys (EcoToys) made of Mater-Bi^{®} type bioplastic having porous consistency and low density (approximately 0.025 g/cm³), a quantity of 10 g of the above-mentioned material (diameter < 1 mm) was transferred to 250 mL flasks containing 30 mL of the culture medium described in example 2. After a phase in the autoclave (15 minutes at 120°C and 105 kPA) and inoculation with 100 µL of aqueous suspension of spores (10⁵ spores/mL) of the strain Beauveria bassiana DSM 1344, cultivated in the same way as in example 1, the flasks were transferred to an incubator at 28°C. After six days of incubation in static conditions, the mycelium was separated from the remaining residues of bioplastic by washing in a funnel separator and subsequent filtering with nylon fabric (cheesecloth for mycology). The mycelium was then dried at 100°C in a ventilated stove for twenty-four hours and then weighed. The quantity of mycelium of the micoinsecticide fungus was evaluated at 1.3 g. The content of the flasks not used for evaluation of the mycelium produced was homogenised with an Ultraturrax type laboratory homogeniser and the aqueous dispersion directly sprayed on tomato plants cv ACE 55 VF (Blumen s.r.l., Milan, Italy) cultivated in Fitotron growth chambers at 28°C with day/night cycles of 14/10 hours. After the transfer of colonies of adult aphids (Myzus persicae) to watermelon seedlings to obtain neanides, groups of 30 fourth-stage neanides were used to uniformly infest the individual tomato plants near to the beginning of flowering. The micoinsecticide preparation was applied the day after the artificial infestation of aphids and after a further four successive days the effectiveness of the preparation was evaluated. The test was performed keeping the individual plants at a distance of 1 m from one another and repeating the test twenty times. The results obtained indicated an effectiveness of the preparation equal to a mortality of 95% of the total individuals counted and 4% in the non-treated control.

### Example 6

Use of articles made of Mater-Bi^{®} type bioplastic having porous consistency and low density for the microbiological production of lactic acid.

The same procedure as the one indicated in example 1 was repeated, replacing the 2.5 g of crushed material from bags with 1 g of crushed material (< 1 mm) obtained from toys made of bioplastic (with porous consistency and low density) based on Mater-Bi^{®} bioplastic. Using the same technique and incubation times, 330 mg of laevorotatory lactic acid were obtained per gram of bioplastic material added.

### Example 7

Use of Mater-Bi^{®} bioplastic toy items (made of Mater-Bi^{®} type bioplastic having porous consistency and low density) for microbiological production of the enzyme alpha amylase.

The same solid-state fermentation procedure as described in example 5 was used for production of the enzyme alpha amylase by the fungus Aspergillus oryzae ATCC 12892. A volume of 200 µL of aqueous suspension of spores (10⁸ spores/mL) of the fungus, obtained as in example 1, was used to inoculate the substrate (10 g of pulverised fragments in 30 mL of minimal culture medium). The flasks were incubated at 35°C in static conditions. After six days of incubation, the mass was transferred to 250 mL bottles containing 150 mL of a sterile aqueous solution containing Tween 80 (0.1%). The bottles were stirred for two hours in a horizontal stirrer operating at 250 rpm. The entire content was then transferred into centrifuge tubes which were centrifuged at 5,000 rpm for fifteen minutes at 4°C. Portions of supernatant were used for the enzymatic assay in a solution consisting of 1.5 mL of 0.2% of soluble starch, 1 mL of phosphate buffer 0.1 M and 0.5 of the above-mentioned supernatant. After fifteen minutes of incubation at 40°C, portions of 10 µL were used to determine the concentration of glucose using the enzymatic assay Glucose Liquid produced by Sentinel Diagnostics S.p.A, Milan, Italy. The readings were taken using spectrophotometer operating at 550 mm. The alpha amylase enzymatic activity of the extracts was evaluated considering one unit (U) of enzymatic activity as the quantity of alpha amylase capable of hydrolysing 0.1 mg of starch in one minute and expressed in U/g of dry substrate. The value obtained with the procedure described above was 825 U/g of dry material.

## Claims

1. Method for microbial production, in particular of organic acids, other molecules and bioactive substances, from bioplastic materials, in particular comprising micro organisms and material made of bioplastic; the method provides that material made of bioplastic is used by a fungus Rhizopus oryzae and subsequently by an entomopathogenic bacterium Bacillus thuringiensis subsq. Kurstaki.

2. Method as claimed in claim 1, wherein the bioplastic comprises polylactic acid.

3. Method as claimed in claim 1 or 2, wherein the entomopathogenic bacterium Bacillus thuringiensis subsq. Kurstaki is the entomopathogenic bacterium Bacillus thuringiensis subsq. Kurstaki strain HD1.

4. Method as claimed in one of the preceding claims, wherein the fungus Rhizopus oryzae is selected from the group consisting of: Rhizopus oryzae CCM 8116, Rhizopus oryzae MB1 and a combination thereof.

5. Method as claimed in one of the preceding claims, wherein the fungus Rhizopus oryzae is Rhizopus oryzae CCM 8116.

6. Method as claimed in one of the preceding claims, wherein the material made of bioplastic consists of bottles made of pulverised bioplastic.

7. Method as claimed in one of the preceding claims, wherein toxins are obtained, in particular Cry1Ab/Cry1Ac toxins and parasporal crystals.

8. Method for microbial production, in particular of organic acids, other molecules and bioactive substances, from bioplastic materials, in particular comprising micro organisms and material made of bioplastic; the method comprises a phase during which the fungus Beauveria bassiana DSM 1344 uses material made of bioplastic, in particular at low density, to develop biomass of the same.

9. Method as claimed in claim 8, wherein the bioplastic comprises Mater-Bi^{®}; in particular the material made of bioplastic consists of toy items (made of Mater-Bi^{®} type bioplastic having porous consistency and low density).

10. Method for microbial production, in particular of organic acids, other molecules and bioactive substances, from bioplastic materials, in particular comprising micro organisms and material made of bioplastic; the method comprises a phase during which material made of bioplastic is used by the fungus Aspergillus niger CCM 8189 to obtain other substances, in particular via a direct fermentation process.

11. Method as claimed in claim 10, wherein the bioplastic comprises Mater-Bi^{®}; in particular the material made of bioplastic consists of bioplastic bags.

12. Method as claimed in claim 10 or 11, wherein material made of bioplastic is used by the fungus Aspergillus niger CCM 8189 for the production of citric acid.

13. Method for microbial production, in particular of organic acids, other molecules and bioactive substances, from bioplastic materials, in particular comprising micro organisms and material made of bioplastic; the method comprises a phase during which material made of bioplastic is used by the fungus Rhizopus oryzae to obtain, in particular via a direct fermentation process, other substances, in particular lactic acid.

14. Method as claimed in claim 13, wherein the material made of bioplastic is selected from the group consisting of: bottles made of pulverised bioplastic, bags made of fragmented bioplastic, in particular with dimensions of less than 2 mm, and a combination thereof; the fungus Rhizopus oryzae is chosen from the group consisting of: Rhizopus oryzae CCM 8116, Rhizopus oryzae MB1 and a combination thereof.

15. Method as claimed in claim 13 or 14, wherein the bioplastic is selected from the group consisting of: polylactic acid, bioplastic obtained from starch (in particular, Mater-Bi^{®}) and a combination thereof; the fungus Rhizopus oryzae is Rhizopus oryzae CCM 8116.
